# EUROPEAN PATENT APPLICATION

(11) **EP 4 140 316 A1**
(43) Date of publication of application: **01.03.2023**
(21) Application number: 21216111.1
(22) Date of filing: 20.12.2021
(51) Int. Cl.: A23L 21/25

(54) **STANDARDIZED HONEY**

(30) Priority: 23.08.2021 US 202163259952 P; 24.08.2021 US 202163259965 P; 10.09.2021 US 202163360193 P
(71) Applicant: Fenzl, Mark Edward, Sarasota, FL 34232 (US)
(72) Inventor: Fenzl, Mark Edward, Sarasota, FL 34232 (US)
(74) Representative: Sach, Greg Robert

(57) **Abstract**

A new means of creating a standardized honey that can be used for a variety of uses. One example is a honey product that protects the stomach and inhibits *H. Pylori.* This product has a consistent minimal antimicrobial effect. The honey may be standardized to pH, organic acid level, mineral levels, aldehyde levels, and polyphenol levels. Molasses or natural syrup may be substituted for honey. Other variations are honey that is supplemented with molasses, natural syrup, standardized molasses and / or standardized natural syrup.

## Description

### CROSS-REFERENCE TO RELATED APPLICATIONS

This application claims the benefit of U.S. Provisional Application 63/259,952, filed on August 23, 2021. The entire disclosure of the above application is incorporated herein by reference.

This application claims the benefit of U.S. Provisional Application 63/259,965, filed on August 24, 2021. The entire disclosure of the above application is incorporated herein by reference.

This application claims the benefit of U.S. Provisional Application 63/360,193, filed on September 10, 2021. The entire disclosure of the above application is incorporated herein by reference.

### FIELD

The present application relates to a new means of creating a standardized honey that can be used for multiple purposes. This product has a consistent minimal antimicrobial effect. One use of this patent includes a standardized antiulcer honey supplement. The honey may be standardized to pH, organic acid levels, mineral levels, aldehydes, and polyphenols. Molasses and natural syrups can be standardized and may be added or substituted for honey. Other uses include food, nutritional supplement, wound honey, burn honey, animal feed, topical preservative, and cosmetic preservative.

### BACKGROUND

The present invention relates a discovery that has been made that adding acids, minerals, aldehydes, and / or polyphenols at a standardized level to honey, molasses, or syrup may create a standardized antimicrobial product. Honey, molasses, and natural syrups contains organic acids, minerals, aldehydes, and polyphenols, but adding these at a consistent level will allow the manufacturer to produce a product with a consistent antimicrobial level of efficacy with each use of each product.

Honey is used for a variety of purposes including as a food, in foods, as a nutritional supplement, in cosmetics, for topical applications, and as a wound dressing. Honey contains a wide variety of beneficial vitamins and minerals that can be standardized to provide a consistent natural form of nutrition. Standardizing honey that is used in foods may provide an antimicrobial benefit to prevent microbial contamination and spoilage that can cost time and money for distributors and markets. Likewise standardized honey can be used in cosmetics to prevent microbial contamination for cosmetics or other topical applications such as shampoos. Honey is used in medicine as a wound dressing and using standardized honey may strengthen the antimicrobial qualities of the honey and provide a more consistent product. There may be a variety of other uses for honey in the future. Molasses, such as blackstrap molasses, or syrup, such as date syrup, can be used as a substitute for honey.

Medical honey is considered to be standardized, but it is standardized according to methylglyoxal content, filtration, and irradiation sterilization. This invention instead standardizes the micronutrient content of the final product.

There are a variety of syrups that have a natural antimicrobial effect including, but not limited to, barley syrup, sorghum syrup, and fruit syrups. These natural syrups have a pH less than 7 and contain sugars, organic acids, polyphenols, minerals, and aldehydes that can be standardized. A person well versed in food products may make an applicable addition to the product within the scope of this patent.

Acidity and water activity levels have an effect on antimicrobial efficacy of honey, molasses, and syrups. Two ways a product is protected from microbial spoilage are water activity level and acidity. Products with low water activity level do not have enough water for bacteria, yeast, or fungus reproduce and cause damage. Acidity or low pH damages microbes, preventing them from damaging the product. Adding ingredients with low water activity and acidity, such as lactic acid, may further protect the product. Ingredients that lower water activity and ingredients that lower the pH may be added to the product as a part of this patent. A person well versed in food products may make an applicable addition to the product within the scope of this patent.

Honey, molasses, and natural syrups contain natural minerals. These minerals are necessary for bodily function and healing. Some minerals also have antimicrobial effects that protect a product from contamination. The minerals may be standardized within the product.

There are a variety of polyphenols found in honey. These polyphenols have natural antimicrobial properties. Standardizing these polyphenols may standardize the antimicrobial level of the product.

There are different polyphenols naturally found in honey that vary by type of honey. Examples of polyphenols found in buckwheat honey are noted in the table below. These polyphenols have antioxidant and antimicrobial properties.

**Table 1.**

| **Polyphenol** | **Range in micrograms per 100 grams of honey** |
|---|---|
| Apigenin | 34 - 80 |
| Caffeic Acid | 26 - 82 |
| Chlorogenic Acid | 51 - 90 |
| Coumaric Acid | 250 - 780 |
| Ferulic Acid | 66 - 166 |
| Galagin | 16 - 26 |
| Hydroxybenzoic Acid | 189 - 410 |
| Kaempferol | 77 - 179 |
| Myricetin | 13 - 42 |
| Protocatechic Acid | 39 - 83 |
| Quercetin | 22 - 450 |

There are different minerals found in honey that vary by type of honey. Examples of minerals found in honey are noted in the table below. These have nutritional and antimicrobial value.

**Table 2.**

| **Mineral** | **Range in milligrams per Kilograms of honey** |
|---|---|
| Potassium | 225 - 439 |
| Magnesium | 31-74 |
| Sodium | 76 - 200 |
| Calcium | 46 - 98 |
| Manganese | 0-1 |
| Iron | 3-16 |
| Cadmium | trace |
| Nickel | trace |
| Copper | trace |
| Lead | trace |
| Chromium | trace |
| Zinc | 1-4 |
| Cobalt | trace |

One example of a standardized honey, that does not limit this patent application, is a honey standardized to a pH between 2 and 7 with lactic acid, quercetin at a concentration between 49 parts per million and 50,001 parts per million, citral at a concentration between 49 and 50,001 parts per million, and iron at a concentration between 49 parts per million and 50,001 parts per million.

Variations that do not limit this patent are when molasses, natural syrup, standardized molasses, and /or standardized natural syrup are added to the above product. Other variations, that do not limit this patent, include the above product in which honey is replaced with molasses or natural syrup.

Lactic acid increases acidity, which has a synergistic antimicrobial effect with several of the ingredients. Lactic acid also supports healthy gut bacteria that inhibit *H*. pylori bacteria. Quercetin is bactericidal of antibiotic-resistant bacteria at a concentration of 5,000 parts per million and has a protective effect on the lining of the stomach. Citral is bactericidal of *H*. pylori, inhibits bacteria, kills mold, and kills fungi. Iron rebuilds red blood cells for those with blood loss associated with ulcers and has a synergistic antimicrobial effect with honey.

This particular product protects the stomach lining, promotes healthy bacteria in the gut, and kills *H*. pylori bacteria. This unique patent creates a honey product with a standard antimicrobial level that can be used for a variety of uses. Other uses for this product include but are not limited to nutritional food, food flavoring, preservative, animal feed, skin ulcer honey, wound honey, and burn honey.

A person knowledgeable in food science may substitute ingredients within this patent to make a similar product to strengthen one of the potential uses or create a more cost efficient product.

### SUMMARY OF THE INVENTION

This invention is an improved standardized honey product using organic acid(s), mineral(s), aldehydes(s), and polyphenols. One example of a standardized honey product, that does not limit this patent application, is an antiulcer honey that has been standardized to a pH between 2.5 and 6 with lactic acid, standardized with quercetin at a concentration between 99 parts per million and 30,001 parts per million, standardized with citral at a concentration between 99 and 30,001 parts per million, and standardized with iron at a concentration between 99 parts per million and 30,001 parts per million. Variations include standardized molasses, standardized natural syrup, molasses and / or natural syrup added to the above product or honey being replaced with molasses or natural syrup. Other uses for a multiuse product within the scope of this patent include food, nutritional supplement, wound honey, burn honey, skin ulcer honey, topical product preservative, and cosmetic preservative.

### DETAILED DESCRIPTION

One example of a standardized honey product, that does not limit this patent application, is a stomach antiulcer nutraceutical that has been standardized to a pH of four or lower using lactic acid, standardized with quercetin at a concentration of at least 5,000 parts per million, standardized with citral at a concentration of least 500 parts per million, and standardized with iron at a concentration of at least 500 parts per million. Two tablespoons can be consumed twice a day for a beneficial effect. This product may also be used as a wound honey, burn honey, sunburn honey, skin ulcer honey, or mucosal ulcer honey.

Variations, that do not limit this patent, include adding standardized molasses, standardized natural syrup, molasses and / or syrup to the above product. Other variations covered by this patent include the same product with molasses or natural syrup replacing honey.

The low pH caused by lactic acid with citral have a synergistic antimicrobial effect against *H*. *Pylori* found in stomach ulcers. Citral and honey have a synergistic antimicrobial effect against *H. Pylori.* Lactic acid and the hydrogen peroxide produced by honey have a synergistic effect. Iron has a synergistic antimicrobial effect with honey and is nutritionally necessary for reversing anemia caused by stomach ulcers. Quercetin protects the stomach lining against ulcers. Lactic acid supports probiotic bacteria found in honey that inhibits *H. pylori.*

A person knowledgeable in medical sciences may substitute an appropriate ingredient within the scope of this patent. Other uses for this product includes but are not limited to medical device preservative, wound product, first aid product, animal feed, food preservatives, and cosmetic preservatives.

## Claims

1. Honey or molasses that is standardized using at least two of an organic acid, a mineral, an aldehyde, and a polyphenol.

2. A honey product in claim 1 that is standardized with lactic acid, iron, citral, and quercetin.

3. A honey product in claim 1 that is standardized to a pH between 2 and 6.

4. A honey product in claim 1 that is standardized with at least one mineral at a concentration between 49 and 50,001 parts per million.

5. A honey product in claim 1 that is standardized with at least one aldehyde at a concentration between 49 and 50,001 parts per million.

6. A honey product in claim 1 that is standardized with polyphenols at a concentration between 49 and 50,001 parts per million.

7. A honey product in claim 1 that is standardized with a least one organic acid and at least one polyphenol.

8. A honey product in claim 1 that is standardized with at least one organic acid and at least one mineral.

9. The process of adjusting of at least two of an acid, a mineral, an aldehyde, and a polyphenol in honey or molasses to standardize the concentrations in the product.

10. The process in claim 9 of using a lactic acid to standardize molasses.

11. The process in claim 9 of using iron to standardize molasses.

12. The process in claim 9 to use a quercetin to standardize molasses.

13. An ulcer syrup, wound syrup, burn syrup, medical syrup, first aid syrup, or syrup preservative standardized with at least one of an organic acid and an aldehyde.

14. A product in claim 13 in which syrup is standardized at a pH between 2 and 6.

15. A product in claim 13 in which citral is standardized at a concentration between 49 parts per million and 50,000 parts per million.
